# EUROPÄISCHE PATENTSCHRIFT

(11) **EP 1 449 499 B1**
(45) Veröffentlichungstag und Bekanntmachung des Hinweises auf die Patenterteilung: **22.11.2006**
(21) Anmeldenummer: 04001144.7
(22) Anmeldetag: 21.01.2004
(51) Int. Cl.: A61F 2/18, A61F 2/46

(54) **Vorrichtung zur Ermittlung der Länge einer Mittelohrprothese**
Device for determing the length of a middle ear prosthesis
Dispositif pour déterminer la longueur d'une prothèse de l'oreille moyenne

(30) Priorität: 21.02.2003 DE 20302850 U
(43) Veröffentlichungstag der Anmeldung: 25.08.2004
(73) Patentinhaber: Heinz Kurz GmbH Medizintechnik, 72144 Dusslingen (DE)
(72) Erfinder: Kurz, Heinz, 72144 Dusslingen (DE); Steinhardt, Uwe, 72145 Hirrlingen (DE)
(74) Vertreter: Möbus, Daniela

(56) Entgegenhaltungen:
- EP-A- 1 181 907
- DE-U1- 20 014 659
- FR-A- 2 785 033
- US-A- 4 921 498
- US-A- 6 113 639

## Beschreibung

Die Erfindung betrifft eine Vorrichtung zur Ermittlung der Länge einer Mittelohrprothese.

Um bei ganz oder teilweise fehlenden Gehörknöchelchen des menschlichen Mittelohrs den Schall vom Trommelfell zum Steigbügel oder zur Steigbügelfußplatte, die das Mittelohr vom Innenohr trennt, zu übertragen, werden Gehörknöchelchenprothesen verwendet. Da die anatomischen Gegebenheiten des Ohres bei verschiedenen Menschen in gewissen Grenzen unterschiedlich sind, ist es notwendig, eine Gehörknöchelchenprothese zu verwenden, deren Abmessung an die speziellen Gegebenheiten des Ohres, bei dem sie eingesetzt werden soll, angepasst ist. Es muss hier insbesondere der Abstand zwischen Trommelfell und der Steigbügelfußplatte bei einer Totalrekonstruktion oder der Abstand zwischen Trommelfell und Steigbügelköpfchen bei einer Partialrekonstruktion bestimmt werden.

Bisher werden dazu Prothesenattrappen in unterschiedlichen Größen, insbesondere mit unterschiedlichen Längen, die aus Edelstrahl hergestellt sind, verwendet. Die Prothesenattrappen werden bei der Operation in das Mittelohr eingeführt, bis die in der Größe stimmende Prothesenattrappe gefunden wurde. Nach der Bestimmung der Größe werden diese Prothesenattrappen in der Klinik in der Regel mittels Dampfsterilisation wieder sterilisiert und stehen somit für eine erneute Größenbestimmung zur Verfügung.

Die Prothesenattrappen sind naturgemäß recht klein. Eine Sterilisation ist daher in ihrer Handhabung relativ schwierig. Um eine ausreichende Sterilisation zu erzielen, muss vorher ein genau definiertes Reinigungsverfahren durchgeführt werden. Weil die Prothesenattrappen so klein sind, besteht auch das Risiko, dass sie bei der Sterilisation oder im Zuge der Vorreinigung verloren gehen können. Da die Prothesenattrappen aus Gründen der Sterilisierbarkeit und Haltbarkeit aus Metall, insbesondere Edelstahl, hergestellt werden, sind sie recht schwer. Das bedeutet, dass sie bei ihrer versuchsweisen Platzierung im Mittelohr umkippen können, so dass eine korrekte Ermittlung der Größe der passenden Prothese viel Geschick verlangt.

Das Dokument EP 1 181 907 A1 zeigt eine Gehörknöchelchenprothese, und FR-A-2 785 033 beschreibt eine Vorrichtung zum Manipulieren eines kleinen Implantats.

Der Erfindung liegt die Aufgabe zugrunde, eine Vorrichtung zur Ermittlung der Länge einer Mittelohrprothese anzugeben, bei der die Prothesenattrappen in leicht zu handhabender Weise zur Verfügung gestellt werden und bei der auch keine Sterilisation notwendig ist.

Die gestellte Aufgabe wird erfindungsgemäß durch die Vorrichtung zur Ermittlung der Länge einer Mittelohrprothese, die die im Hauptanspruch aufgeführten Merkmale aufweist, gelöst. Die Unteransprüche geben bevorzugte Weiterbildungen an.

Bei der erfindungsgemäßen Vorrichtung zur Ermittlung der Länge einer Mittelohrprothese sind verschieden große Prothesenattrappen an einem Basisteil befestigt. Sie werden durch diese Anordnung in übersichtlicher Weise dem Arzt präsentiert und können nicht verloren gehen. Die Vorrichtung umfasst ferner einen Applikator, mit dem die Prothesenattrappen bei der Operation in sicherer Weise zur Ermittlung der Länge der passenden Mittelohrprothese in das Mittelohr eingeführt werden können.

Das Basisteil, an dem die Prothesenattrappen befestigt sind, kann verschiedene Formen und Größen annehmen. Runde, vieleckige und baum- oder stabförmige Ausgestaltungen haben sich dabei in der Praxis als besonders vorteilhaft erwiesen. Die Größe des Basisteils richtet sich nach der Anzahl der Prothesenattrappen und ihrer Befestigungsweise. In der Praxis hat sich ein Größe von 2,5 cm bewährt.

Das Basisteil kann aus verschiedenen Materialien bestehen. Kunststoff hat sich als ein steriles und leicht und kostengünstig beispielsweise durch einen Spritzguss zu verarbeitendes Material für das Basisteil bewährt.

Die Prothesenattrappen sind mittels dünner Stege am Basisteil befestigt. Diese Stege können von Hand unter Verwendung von beispielsweise einer Pinzette leicht gebrochen werden, um die Prothesenattrappe vom Basisteil zu trennen und am Applikator zu befestigen.

Auch bei den Prothesenattrappen ist es sinnvoll, wenn sie aus Kunststoff hergestellt sind. Sie können dann beispielsweise zusammen mit dem Basisteil in einem Spritzgussverfahren leicht und kostengünstig hergestellt werden. Kunststoff ist im Vergleich zu Metall, aus dem bisher bekannte Prothesenattrappen hergestellt waren, ein relativ leichtes Material, so dass die Prothesenattrappen somit auch ein geringeres Gewicht aufweisen. Beim versuchsweisen Einbringen der Prothesenattrappen ins Mittelohr besteht somit eine kleinere Wahrscheinlichkeit, dass die Prothesenattrappen umfallen, so dass die Ermittlung der passenden Größe in zuverlässigerer Weise erfolgen kann. Sollte der Kunststoff für die Prothesenattrappe nicht die benötigte Festigkeit liefern, so kann die Prothesenattrappe zur Versteifung einen Kern aus Metall erhalten, oder man kann durch den gezielten Einsatz von Metallkernen eine gewünschte Gewichtsverlagerung zum besseren Handling erzeugen.

Der Applikator besteht vorzugsweise aus einem Stab, der an seinem einen Ende einen zangenförmigen Aufnahmeteil zum Aufnehmen der Prothesenattrappe aufweist. Er kann aus verschiedenen Materialien, insbesondere aus Metall oder Kunststoff, hergestellt sein. Um die Prothesenattrappe sicher zu halten, ist es vorteilhaft, wenn der zangenförmige Aufnahmeteil federnd ausgebildet ist. Es ist aus diesem Grund auch von Vorteil, wenn die Innenseite des zangenförmigen Aufnahmeteils aufgeraut ist.

Die einzelnen Teile der erfindungsgemäßen Vorrichtung, also das Basisteil mit den daran befestigten verschieden langen Prothesenattrappen und der Applikator, sind vorzugsweise steril verpackt und stehen somit, ohne dass eine vorherige Sterilisation erfolgen muss, sofort zur Anwendung bereit. Nachdem die richtige Länge für die spätere Prothese ermittelt wurde, werden die Prothesenattrappen entsorgt, wodurch man sich die kritische Reinigung und Sterilisation erspart und somit das Risiko einer Infektübertragung ausräumt.

Ein Ausführungsbeispiel einer erfindungsgemäß ausgebildeten Vorrichtung zur Ermittlung der Länge einer Mittelohrprothese wird nachfolgend anhand der beiliegenden Zeichnung erläutert. In den Zeichnungen sind gleiche Elemente in allen Zeichnungsfiguren mit den gleichen Bezugszahlen gekennzeichnet.

Es zeigen:
- Fig. 1: einen perspektivische Ansicht einer Ausführungsform des Basisteils der erfindungsgemäßen Vorrichtung mit daran befestigten Prothesenattrappen; und
- Fig. 2: eine perspektivische Teilansicht einer Ausführungsform des Applikators der erfindungemäßen Vorrichtung mit einer darin aufgenommenen Prothesenattrappe.

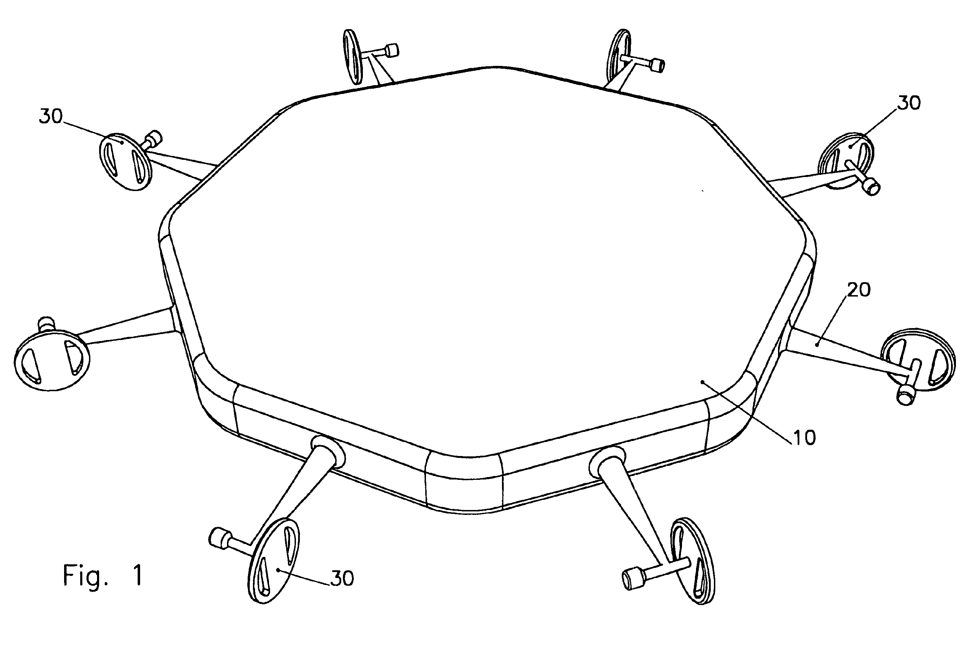

Die Figur 1 zeigt in perspektivischer Darstellung eine Ausführungsform des Basisteils der erfindungsgemäßen Vorrichtung zur Ermittlung der Länge einer Mittelohrprothese mit daran befestigten Prothesenattrappen. Das Basisteil 10 ist hier in Form eines gerundeten Achtecks ausgebildet. An jeder seiner Seiten ist über einen Steg 20 eine Prothesenattrappe 30 befestigt.

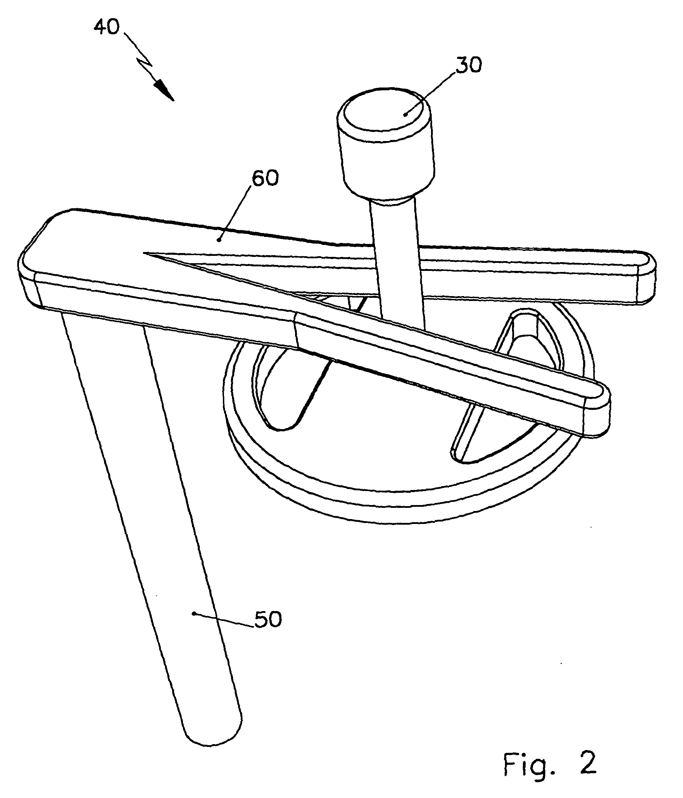

Die Figur 2 zeigt in einer perspektivischen Teilansicht eine Ausführungsform des Applikators 40 der erfindungsgemäßen Vorrichtung zur Ermittlung der Länge einer Mittelohrprothese. Der Applikator 40 besteht aus einem Stab 50 und einem zangenförmigen Aufnahmeteil 60. Im Aufnahmeteil 60 ist eine Prothesenattrappe 30 untergebracht und kann so leicht mit dem Applikator versuchsweise in das Mittelohr eingebracht werden, um die passende Größe der späteren Prothese zu ermitteln.

### Bezugszeichenliste

- 10: Basisteil
- 20: Steg
- 30: Prothesenattrappe
- 40: Applikator
- 50: Stab
- 60: Aufnahmeteil

## Patentansprüche

1. Vorrichtung zur Ermittlung der Länge einer Mittelohrprothese, **dadurch gekennzeichnet, dass** sie ein Basisteil (10), an dem verschieden lange Prothesenattrappen (30) befestigt sind, und einen Applikator (40), mit dem diese Prothesenattrappen (30) nach dem Lösen vom Basisteil (10) während einer Operation in das Mittelohr eingeführt werden, umfasst.

2. Vorrichtung nach Anspruch 1, **dadurch gekennzeichnet, dass** das Basisteil (10) eine runde, eine eckige, eine baumförmige oder eine stabförmige Form aufweist.

3. Vorrichtung nach Anspruch 1 oder 2, **dadurch gekennzeichnet, dass** das Basisteil (10) aus Kunststoff besteht.

4. Vorrichtung nach einem der vorhergehenden Ansprüche, **dadurch gekennzeichnet, dass** das Basisteil (10) eine Größe von ca. 2,5 cm aufweist.

5. Vorrichtung nach einem der vorhergehenden Ansprüche, **dadurch gekennzeichnet, dass** die Prothesenattrappen (30) mittels dünner und manuell brechbarer Stege (20) mit dem Basisteil (10) verbunden sind.

6. Vorrichtung nach einem der vorhergehenden Ansprüche, **dadurch gekennzeichnet, dass** die Prothesenattrappen (30) aus Kunststoff hergestellt sind.

7. Vorrichtung nach Anspruch 6, **dadurch gekennzeichnet, dass** die Prothesenattrappen (30) einen Kern aus Metall aufweisen.

8. Vorrichtung nach einem der vorhergehenden Ansprüche, **dadurch gekennzeichnet, dass** der Applikator (40) aus einem Stab (50) besteht, der an einem Ende einen zangenförmige Aufnahmeteil (60) zum Aufnehmen einer Prothesenattrappe (30) aufweist.

9. Vorrichtung nach Anspruch 8, **dadurch gekennzeichnet, dass** der Applikator (40) aus Metall oder Kunststoff hergestellt ist.

10. Vorrichtung nach Anspruch 8 oder 9, **dadurch gekennzeichnet, dass** der zangenförmige Aufnahmeteil (60) federnd ausgebildet ist.

11. Vorrichtung nach einem der Ansprüche 8 bis 10, **dadurch gekennzeichnet, dass** die Innenseite des zangenförmigen Aufnahmeteils (60) aufgeraut ist.

12. Vorrichtung nach einem der vorhergehenden Ansprüche, **dadurch gekennzeichnet, dass** die einzelnen Teile der Vorrichtung in einer Verpackung steril verpackt sind.

## Claims

1. Device for determining the length of a middle ear prosthesis, **characterized in that** it comprises a base part (10) to which dummy prostheses (30) of different lengths are attached and an applicator (40) with which these dummy prostheses (30) after having been detached from the base part (10) are introduced into the middle ear during an operation.

2. Device according to Claim 1, **characterized in that** the base part (10) has a round, angular, treelike or bar-shaped form.

3. Device according to Claim 1 or Claim 2, **characterized in that** the base part (10) consists of plastic material.

4. Device according to any one of the preceding claims, **characterized in that** the base part (10) is approx. 2.5 cm in size.

5. Device according to any one of the preceding claims, **characterized in that** the dummy prostheses (30) are joined to the base part (10) by thin, manually breakable webs or stems (20).

6. Device according to any one of the preceding claims, **characterized in that** the dummy prostheses (30) are made of plastic material.

7. Device according to Claim 6, **characterized in that** the dummy prostheses (30) have a metal core.

8. Device according to any one of the preceding claims, **characterized in that** the applicator (40) consists of a rod (50) with a tweezerlike holder part (60) at one end for holding a dummy prosthesis (30).

9. Device according to Claim 8, **characterized in that** the applicator (40) is made of metal or plastic material.

10. Device according to Claim 8 or Claim 9, **characterized in that** the tweezerlike holder part (60) is springy.

11. Device according to any one of Claims 8 to 10, **characterized in that** the inside of the tweezerlike holder part (60) is roughened.

12. Device according to any one of the preceding claims, **characterized in that** the individual parts of the device are packed in sterile packs.

## Revendications

1. Dispositif pour déterminer la longueur d'une prothèse de l'oreille moyenne, **caractérisé en ce qu'**il comprend une partie de base (10), à laquelle sont fixées des prothèses factices (30) de diverses longueurs, et un applicateur (40) avec lequel ces prothèses factices (30), après les avoir détachées de la partie de base (10), sont introduites dans l'oreille moyenne pendant une opération.

2. Dispositif selon la revendication 1, **caractérisé en ce que** la partie de base (10) présente une forme ronde, polygonale, arborescente ou de barreau.

3. Dispositif selon la revendication 1 ou 2, **caractérisé en ce que** la partie de base (10) est réalisée en matière plastique.

4. Dispositif selon une des revendications précédentes, **caractérisé en ce que** la partie de base (10) possède une dimension d'environ 2,5 cm.

5. Dispositif selon une des revendications précédentes, **caractérisé en ce que** les prothèses factices (30) sont reliées à la partie de base (10) au moyen de liaisons minces et cassables manuellement (20).

6. Dispositif selon une des revendications précédentes, **caractérisé en ce que** les prothèses factices (30) sont réalisées en matière plastique.

7. Dispositif selon la revendication 6, **caractérisé en ce que** les prothèses factices (30) possèdent un noyau en métal.

8. Dispositif selon une des revendications précédentes, **caractérisé en ce que** l'applicateur (40) est constitué d'une barre (50) qui comporte à une extrémité une partie réceptrice en forme de pince (60) pour recevoir une prothèse factice (30).

9. Dispositif selon la revendication 8, **caractérisé en ce que** l'applicateur (40) est réalisé en métal ou en matière plastique.

10. Dispositif selon la revendication 8 ou 9, **caractérisé en ce que** la partie réceptrice en forme de pince (60) est de conception élastique.

11. Dispositif selon une des revendications 8 à 10, **caractérisé en ce que** le côté intérieur de la partie réceptrice en forme de pince (60) est rugueux.

12. Dispositif selon une des revendications précédentes, **caractérisé en ce que** les parties individuelles du dispositif sont emballées de manière stérile dans un emballage.
